# EUROPÄISCHE PATENTSCHRIFT

(11) **EP 0 566 036 B1**
(45) Veröffentlichungstag und Bekanntmachung des Hinweises auf die Patenterteilung: **14.08.1996**
(21) Anmeldenummer: 93105843.2
(22) Anmeldetag: 08.04.1993
(51) Int. Cl.: A61B 17/12, A44B 11/12, A61B 17/132, A44B 11/06, A44B 11/25

(54) **Abschnürvorrichtung für Körperteile, insbesondere Venenstauer**
Ligaturing device for parts of the human body, in particular tourniquet
Dispositif de ligature pour des parties du corps humain, en particulier tourniquet

(30) Priorität: 11.04.1992 DE 9205067 U
(43) Veröffentlichungstag der Anmeldung: 20.10.1993
(73) Patentinhaber: HOLTSCH Metallwarenherstellung Maria Holtsch, D-65232 Taunusstein (DE)
(72) Erfinder: Holtsch, Peter, W-6204 Taunusstein (DE)
(74) Vertreter: Eisenführ, Speiser & Partner

(56) Entgegenhaltungen:
- EP-A- 0 091 403
- EP-A- 0 196 646
- EP-A- 0 375 901
- WO-A-88/00456
- DE-U- 9 200 574
- GB-A- 2 138 490

## Beschreibung

Die Erfindung betrifft eine Abschnürvorrichtung für Körperteile, insbesondere Venenstauer, mit einem Gehäuse, in dem eine Klemmwippe schwenkbar gelagert ist, welche in einer Klemmstellung mit ihrem einen Ende ein das Gehäuse durchsetzendes Band klemmend gegen eine Gehäusewand drückt und in einer entgegengesetzt gerichteten Lösestellung das Band freigibt, wobei das in der Abschnürlage unter Spannung stehende Band die Klemmwippe in ihre Klemmstellung schwenkt, während ein manueller Druck auf die Klemmwippe diese in die Lösestellung schwenkt,
sowie mit einem lösbaren Rastverschluß zwischen dem anderen Ende der Klemmwippe und einer Kappe am gehäuseseitigen Ende des Bandes.

Das - regelmäßig elastische - Band bildet eine Schlaufe um das abzuschnürende Körperteil. Wird das Band durch Ziehen an seinem freien Ende gespannt, so schwenkt die am gehäuseseitigen Ende auftretende Kraft die Klemmwippe in ihre Klemmstellung, in der sie das Band in seiner Abschnürlage arretiert. Soll nur die Abschnürung beendet werden, ohne die Umschlingung des Körperteils aufzuheben - etwa weil kurz darauf eine neuerliche Abschnürung erforderlich werden könnte -, so genügt ein Fingerdruck auf die Klemmwippe (direkt oder mittels eines Zwischengliedes), um diese in ihre Lösestellung zu schwenken, in der das Band durch das Verschlußgehäuse zurückgleiten kann und die Abschnürspannung aufgehoben wird. Soll hingegen die Arbeit mit der Abschnürvorrichtung gänzlich beendet werden, so wird der Rastverschluß gelöst und auf diese Weise die Umschlingung des Körperteils beendet, ohne daß das Band mit seinem freien Ende durch das Verschlußgehäuse hindurch zurückgezogen werden müßte.

Diese Venenstauer-Ausbildung wie sie beispielsweise aus dem DE-U-92 00 574.8 bekannt ist, hat sich in der Praxis bewährt. Die beiden Funktionen des Band-Spannens bzw. -Lösens einerseits und des Band-Öffnens andererseits erlauben eine gute Anpassung an unterschiedliche Einsatzfälle bei rascher Handhabung der Abschnürvorrichtung. Andererseits erfordert die Trennung der beiden Funktionen entsprechend getrennte Handgriffe für die Betätigung.

Der Erfindung liegt daher die Aufgabe zugrunde, die Handhabung einer Abschnürvorrichtung der eingangs beschriebenen Art zu vereinfachen.

Sie wird erfindungsgemäß dadurch gelöst, daß mindestens ein mechanisches Übertragungsglied vorgesehen ist, welches bei Ausübung eines Betätigungsdruckes zunächst die Klemmwippe in ihre Lösestellung schwenkt und nach der Freigabe des Bandes den Rastverschluß öffnet. Auf diese Weise entsteht eine »Einorganbetätigung« der Abschnürvorrichtung; die Notwendigkeit, unterschiedliche Betätigungen zur Erzielung der beiden Funktionen vorzunehmen, entfällt.

Darüber hinaus ist die Gefahr gebannt, daß ein Öffnen der Bandschlinge am Rastverschluß aus der Abschnürlage des Bandes - also unter Spannung des Bandes - den mit der Abschnürvorrichtung behandelten Patienten erschreckt oder gar verletzt und beispielsweise bei der Blutentnahme zu einer spontanen Veränderung des Blutbildes führt. Vielmehr ist gewährleistet, daß die Bandschlinge nur geöffnet werden kann, wenn zuvor eine noch vorhandene Abschnürspannung im Band aufgehoben wurde.

Die Erfindung läßt sich in unterschiedlicher Weise konstruktiv umsetzen. Eine erste Alternative sieht vor, daß beim Schwenken der Klemmwippe aus ihrer Klemmstellung das Übertragungsglied erst in der Lösestellung der Klemmwippe die Wirkverbindung zum Rastverschluß herstellt und diesen - bei weiterem Schwenken der Klemmwippe bis in eine Öffnungsstellung - öffnet. Dabei kann das Übertragungsglied (bzw. deren mehrere) zwischen die Klemmwippe und den Rastverschluß eingeschaltet oder ein zusätzliches Betätigungsorgan sein, welches auf seinem Betätigungsweg zunächst die Klemmwippe in deren Lösestellung schwenkt und auf dem anschließenden Betätigungsweg die Rastverbindung öffnet.

Bei einer solchen Ausbildung kann ein 'Druckpunkt' den Übergang von der Klemmwippen-Betätigung auf die Rastverschluß-Betätigung markieren, vorteilhaft dadurch, daß die Übertragungsglieder dem Betätigungsdruck auf die Klemmwippe beim Öffnen des Rastverschlusses einen größeren Widerstand entgegensetzen als zur Freigabe des Bandes zu überwinden ist. Dies läßt sich insbesondere dadurch erreichen, daß die Schließkraft mindestens einer Feder, die den Rastverschluß in seine Schließstellung drückt, mit einer größeren Kraftkomponente der Öffnungsbetätigung entgegenwirkt als das Band in seiner Abschnürstellung auf die Klemmwippe im Klemmsinne ausübt.

Eine noch sichere zeitliche Trennung des Rastverschluß-Öffnens vom voraufgehenden Lösen der Bandspannung läßt sich erreichen, wenn eine Sperre die Wirksamkeit der Übertragungsglieder bis zu einer Unterbrechung des Betätigungsdruckes nach der Freigabe des Bandes blockiert. Die Bedienungsperson muß also, um die zweite Stufe der Betätigung - das Öffnen des Rastverschlusses - einzuleiten, das jeweilige Betätigungsorgan kurz vom Betätigungsdruck entlasten; wenn der Betätigungsdruck dann wieder aufgebracht wird, erfolgt die Öffnung der Bandschlinge. In der Zwischenzeit aber konnte sich die Abschnürspannung im Band mit Sicherheit abbauen.

In allen vorstehend erörterten Fällen kann die Druckbetätigung direkt auf die Klemmwippe erfolgen, welche mit dem Übertragungsglied (bzw. den Übertragungsgliedern) in Eingriff steht oder kommt. Es können jedoch auch Zwischenglieder für die Betätigung einerseits der Klemmwippe, andererseits des Rastverschlusses vorgesehen sein, beispielsweise eine mit der Klemmwippe in gelenkiger Wirkverbindung stehende Drucktaste oder auch Schiebetasten, welche bei der Betätigung zunächst mit der Klemmwippe und anschließend mit den Bauteilen der Rastverbindung in Eingriff kommen.

Eine besonders vorteilhafte Ausführungsform der die Betätigungssequenz unterbrechenden Sperre besteht darin, daß die Schwenklager der Klemmwippe im Gehäuse längsverschieblich sind und das Band in seiner Abschnürlage die Klemmwippe gegen die Kraft einer Feder in eine erste Endstellung zieht, in der bei Druckbetätigung der Klemmwippe ein Anschlag deren Schwenkbewegung auf das Erreichen der Lösestellung begrenzt, bis nach Freigabe (und Zugentlastung) des Bandes die Feder die Klemmwippe in ihre zweite Endstellung bewegt, in der sie in ihre Öffnungsstellung geschwenkt werden kann und dabei den Rastverschluß öffnet. Bevorzugt hat dabei der Anschlag einen sich im wesentlichen rechtwinklig erstreckenden Abschnitt, der die Längsbewegung der Klemmwippe unter der Federkraft verhindert (»Fangnest«), bis die Klemmwippe in eine Freigabestellung zurückschwenkt.

Weitere vorteilhafte Ausgestaltungen der Erfindung sind Gegenstand zusätzlicher Unteransprüche; hierauf sei an dieser Stelle verwiesen.

Die Zeichnungen veranschaulichen die Erfindung an mehreren Ausführungsbeispielen, und zwar zeigt:
- Fig. 1: eine Seitenansicht der Abschnürvorrichtung in der Abschnürstellung des Gehäuses;
- Fig. 2: eine der Fig. 1 ähnliche Seitenansicht in der Öffungsstellung;
- Fig. 3: eine perspektivische Explosionsdarstellung wesentlicher Teile des Verschlußgehäuses und des Rastverschlusses;
- Fig. 4: eine teilweise aufgeschnittene Draufsicht auf die in Fig. 2 dargestellte Anordnung;
- Fig. 5: zwei Querschnitte gemäß der Linie V-V in Fig. 4, und zwar a) in der Abschnürstellung (Fig. 1) und b) in der Öffnungsstellung (Fig. 2 und 4);
- Fig. 6: eine Ansicht in Richtung des Pfeiles VI in Fig. 4 (in der Öffnungsstellung);
- Fig. 7: in Draufsicht eine alternative Ausführungsform des Verschlußgehäuses;
- Fig. 8: eine Ansicht in Richtung des Pfeiles 8 in Fig. 7;
- Fig. 9: eine der Fig. 7 ähnliche Darstellung, jedoch in Form eines horizontalen Längsschnittes durch das Verschlußgehäuse und in der Öffnungsstellung;
- Fig. 10: eine Ansicht in Richtung des Pfeiles X in Fig. 9;
- Fig. 11: eine schematische Darstellung in Seitenansicht bzw. mittlerem vertikalen Längsschnitt einer wieder anderen Ausführungsform des Verschlußgehäuses und des Rastverschlusses;
- Fig. 12: eine teilweise geöffnete Draufsicht auf das Verschlußgehäuse und den Rastverschluß in Fig. 11;
- Fig. 13: eine Abschnürvorrichtung mit einer wieder anderen Ausführungsart des erfindungsgemäßen Verschlußgehäuses nebst Rastverschluß, und zwar im mittleren vertikalen Längsschnitt sowie in der Abschnürstellung;
- Fig. 14: eine der Fig. 13 entsprechende Darstellung, jedoch in der Lösestellung;
- Fig. 15: eine den Fig. 13 und 14 ähnliche Darstellung, jedoch in der Öffnungsstellung;
- Fig. 16: in teilweise aufgeschnittener Explosions-Teildarstellung eine abgewandelte Ausführungsform des Sperrmechanismus zum Abfangen der Klemmwippe in der Lösestellung; und
- Fig. 17: eine ausschnittsweise Teildarstellung einer die Klemmwippe betätigenden Drucktaste mit einer Einrichtung zum Erzeugen eines Knackgeräusches beim Bewegen der Klemmwippe in die Lösestellung, und zwar in Draufsicht = a) sowie in schematischer Seiten/Schnitt-Ansicht = b).

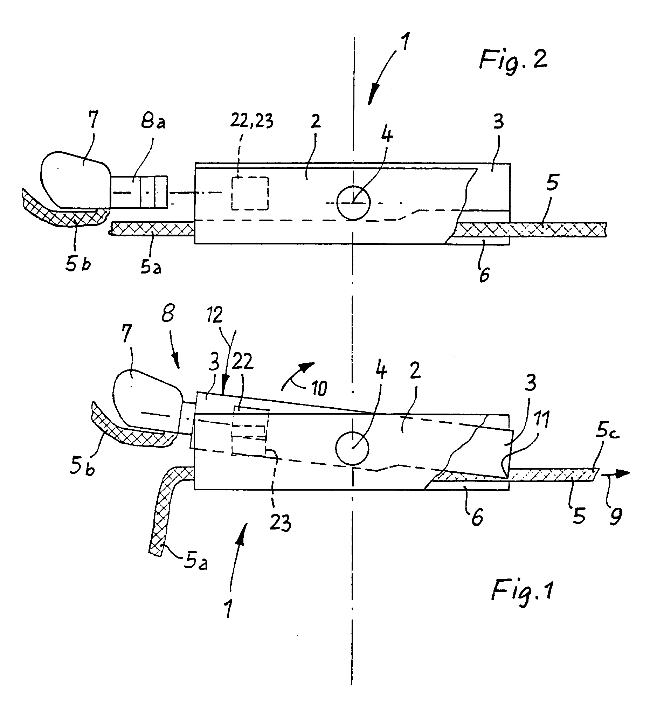

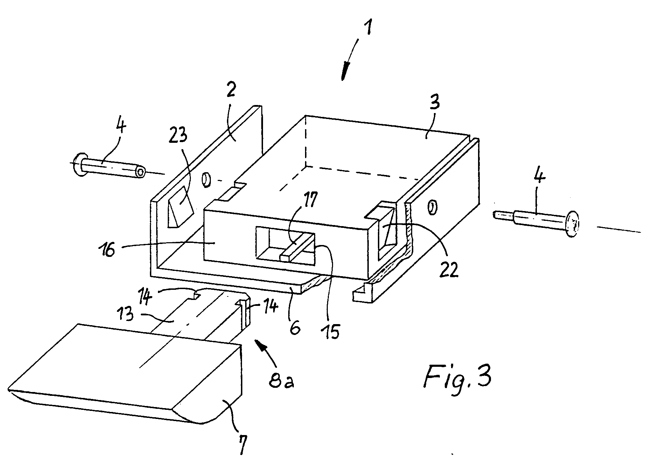

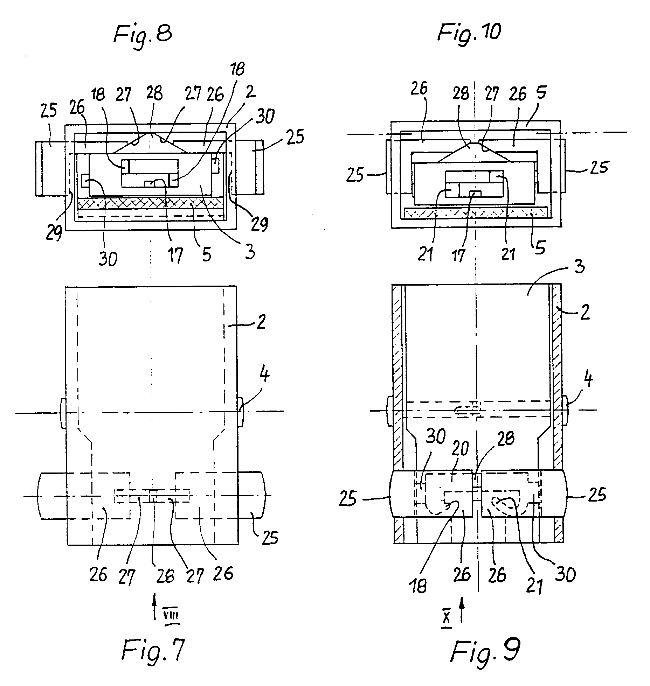

Ein Verschluß 1 hat ein Verschlußgehäuse 2, welches im Querschnitt die Form eines breiten U aufweist (Fig. 3), jedoch zumindest oben auch mindestens teilweise geschlossen sein kann (vgl. Fig. 7 - 10 und 13 - 17). Darin ist etwa mittig eine Klemmwippe 3 mit Hilfe eines aus zwei Teilen bestehenden Stiftes 4 (Fig. 3) schwenkbar gelagert. Ein Abschnürband 5 läuft zwischen dem Bodenabschnitt 6 des Gehäuses 2 und der Klemmwippe 3 hindurch. Es bildet - links in Fig. 1 - eine nur mit den Bandabschnitten 5a und 5b dargestellte Bandschlaufe; das gehäuseseitige Bandende am Abschnitt 5b ist in einer Kappe 7 befestigt, die den einen Partner 8a des im ganzen mit 8 bezeichneten Rastverschlusses trägt. Der andere, in den Fig. 1 und 2 nicht dargestellte Rastverschluß-Partner befindet sich in der Klemmwippe 3. In Fig. 1 ist der Rastverschluß 8 geschlossen, d.h. in der Klemmwippe 3 festgelegt. Das Band 5 wurde an seinem freien Ende 5c in Richtung des Pfeiles 9 durch das Verschlußgehäuse 2 hindurchgezogen, wodurch - infolge der schräg aufwärts gerichteten Abschnürzugkraft im Abschnitt 5b des Bandes 5 - die Klemmwippe 3 in Richtung des Pfeiles 10 nach oben geschwenkt wurde. Demzufolge hat sich ihre Klemmkante 11 in das Band 5 eingegraben und arretiert dies gegenüber einem Rückgleiten entgegen der Pfeilrichtung 9.

Wird aber ein Druck im Bereich und in Richtung des Pfeiles 12 auf die Klemmwippe 3 ausgeübt, so schwenkt diese entgegen dem Pfeil 10 um ihre Achse 4. Die Klemmkante 11 gibt das Band 5 frei, welches infolge der Rückstellkraft im Band selbst sowie im abgeschnürten Körperteil entgegen dem Pfeil 9 durch das Verschlußgehäuse 2 gleiten kann: Die Lösestellung ist erreicht.

In unten noch näher zu beschreibender Weise führt eine weitere Schwenkbewegung der Klemmwippe 3 durch Druck in Richtung des Pfeiles 12 zum Öffnen des Rastverschlusses 8. Die Kappe 7 mit dem Rastpartner 8a am Bandabschnitt 5b und damit dem gehäuseseitigen Ende des Bandes 5 löst sich von der Klemmwippe 3: Die in Fig. 2 dargestellte Öffnungsstellung ist erreicht.

Mit welchen konstruktiven Maßnahmen diese Löse- und Öffnungssequenz des Verschlusses 1 (mit dem Rastverschluß 8) beispielsweise erreicht werden kann, zeigen zunächst die Figuren 3 - 6. Der Rastpartner 8a an der Kappe 7 besitzt eine Zunge 13, welche im Bereich ihres freien Endes zu zwei spiegelsymmetrisch zur Längsachse 19 seitlich auskragenden Haken 14 geformt ist. Die Zunge 13 kann in eine Öffnung 15 in der zugewandten Stirnseite 16 der Klemmwippe 3 eingeführt werden; eine mittlere Führung 17 in der Klemmwippe greift dabei in eine (nicht dargestellte) Nut in der Unterseite der Zunge 13 ein.

Im eingerasteten Zustand, der in den Zeichnungen nicht dargestellt ist, greifen die Haken 14 an der Zunge 13 des Rastpartners 8a hinter Rastnocken 18 an zwei zur Längsachse 19 spiegelsymmetrisch ausgebildeten Schiebegliedern 20, welche querverschieblich in der Klemmwippe 3 geführt sind. Den Rastnocken 18 jeweils gegenüberliegende Federzungen 21 legen sich an den jeweils anderen Haken 14 an und sorgen mit ihrer im Rastzustand durch elastische Auslenkung eingeprägten Federkraft dafür, daß die Schiebeglieder 20 keine ungewollte Querbewegung ausführen, wodurch sich die Rastverbindung zwischen den Rastnocken 18 und den Haken 14 lösen würde. Eine derartige Rastverschluß-Ausbildung ist aus dem DE-U-92 00 574.8 bekannt.

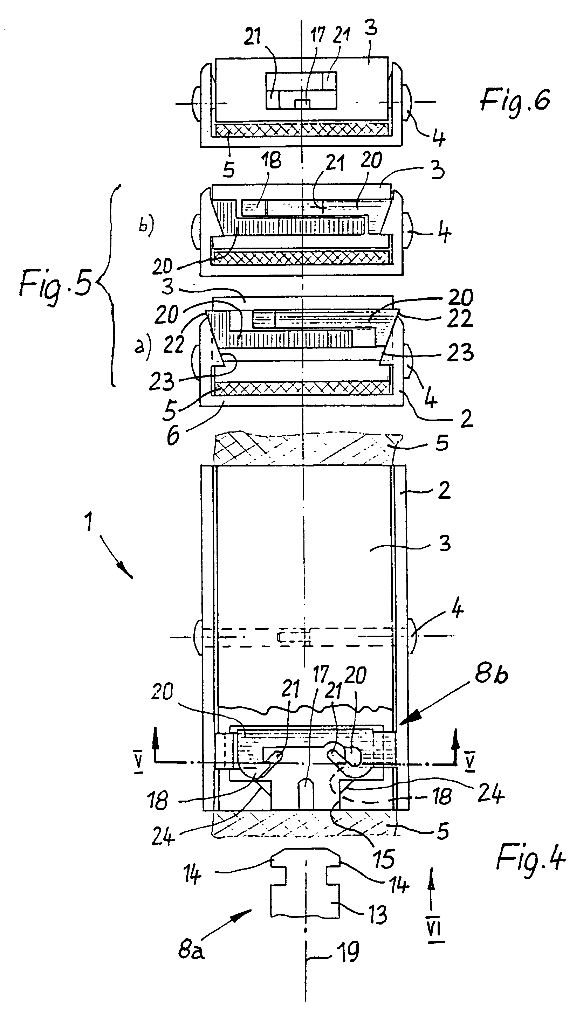

Anders als beim bekannten Rastverschluß laufen die Schiebeglieder 20 jedoch nicht in seitlichen, von außen zugänglichen Druckknöpfen aus, die zum Öffnen des Rastverschlusses, nämlich zum Auseinanderbewegen der Rastnocken 18 entgegen der Kraft der Federzungen 21 dienen. Vielmehr sind sie, wie die Fig. 3 und 5 verdeutlichen, seitlich mit Keilflächen 22 versehen, welche an entsprechend gegengeneigten Keilflächen 23 an den Seitenwänden des Verschlußgehäuses 2 anliegen und mit diesen in Eingriff stehen. Fig. 5 veranschaulicht, daß bei einem Druck auf die Klemmwippe (in Richtung des Pfeiles 12 in Fig. 1) zwangsläufig zunächst die Lösestellung und damit die Freigabe des Bandes 5 durch die Klemmkante 11 der Klemmwippe 3 erreicht wird, ehe anschließend - bei weiterem Druck in Richtung des Pfeiles 12 und damit weiterem Schwenken der Klemmwippe entgegen dem Pfeil 10 - die Keilflächen 23 am Verschlußgehäuse 2 im Zusammenwirken mit den Keilflächen 22 die Schiebeglieder 20 so weit gegeneinander verschoben haben, daß sie aus der in Fig. 5a gezeigten Raststellung in die in Fig. 5b gezeichnete Öffnungsstellung des Rastverschlusses 8 gelangen und die Kappe 7 mit dem Rastpartner 8a aus der Öffnung 15 der Klemmwippe 3 auswerfen.

Die in Fig. 4 erkennbaren Abschrägungen 24 an der der Führung der Schiebeglieder 20 dienenden Ausnehmung in der Klemmwippe 3 verstärken den Eingriff der Rastnocken 18 mit den Haken 14 bei zunehmender Abschnür-Zugkraft im Band 5, damit sichergestellt ist, daß auch bei starker Abschnürkraft kein ungewolltes Lösen des Rastverschlusses 8 erfolgt.

Die Fig. 7 - 10 veranschaulichen ein abgewandeltes Ausführungsbeispiel, bei dem nicht die Klemmwippe betätigt und erst nach Erreichen ihrer - das Band 5 freigebenden - Lösestellung sowie weiterem Schwenken die Öffnungsstellung erreicht wird, in der der Rastverschluß zusätzlich geöffnet ist, vielmehr sind hier seitlich aus dem Verschlußgehäuse 2 mit Betätigungsknöpfen 25 herausragende Schiebetasten 26 vorgesehen. Diese wirken mit schrägen Auflaufflächen 27 auf einen keilförmigen Aufsatz 28, der etwa im Bereich des Pfeiles 12 (Fig. 1) auf der Klemmwippe 3 angebracht ist. Mit senkrechten Flächen 29 wirken die Betätigungsknöpfe 25 ferner auf die seitlichen Stirnflächen 30 der Schiebeglieder 20, bezüglich derer auf die voraufgegangene Beschreibung des Ausführungsbeispiels der Figuren 3 - 6 verwiesen werden kann.

Die Fig. 7 und 8 zeigen - unter weitgehender Weglassung beider Rastpartner 8a und 8b - die Anordnung in der Abschnürstellung, in der die Klemmwippe sich in ihrer Klemmstellung befindet und deshalb die Schiebetasten 26 mit Hilfe des keilförmigen Aufsatzes 28 seitlich herausgeschoben hat. Werden die Betätigungsknöpfe 25 gegeneinandergedrückt, so wird infolge des Abstandes zwischen den Flächen 29 und 30 zunächst nur die Klemmtaste 3 mit Hilfe des keilförmigen Aufsatzes 28 und den Schrägflächen 27 in ihre Lösestellung geschwenkt. Erst dann kommen die Flächen 29, 30 zur Anlage, und weiterer Schiebedruck auf die Betätigungsknöpfe 25 führt zum Öffnen des Rastverschlusses; dieser Zustand ist in den Fig. 9 und 10 dargestellt bzw. angedeutet.

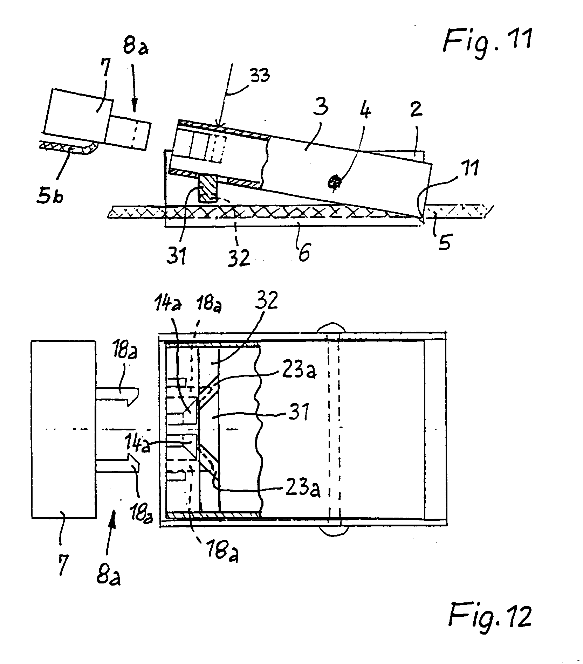

Eine wiederum andere Anordnung veranschaulichen die Fig. 11 und 12 in schematischer Darstellung. Hier ist ein Bock 31 mittig auf einer Brücke 32 befestigt, welche oberhalb des Bandes 5 zwischen den Seitenwänden des Verschlußgehäuses 2 und an diesen befestigt angeordnet ist. Der Bock 31 hat doppelt schräg verlaufende Seitenwände 23a; diese divergieren von oben nach unten (Fig. 12) sowie von vorn nach hinten. An die Seitenwände 23a legen sich, wenn im Rastzustand die einwärts weisenden Haken 18a des Rastpartners 8a über die Rastnocken 14a in der Klemmwippe 3 greifen, die entsprechend schrägen Außenflächen der Haken 14a an, so daß - wenn die Klemmwippe 3 durch Druck in Richtung des Pfeiles 33 im Löse- und Öffnungssinne um die Achse 4 geschwenkt wird - nach dem Lösen der Spannung im Band 5 die Haken 18a federnd auswärts gedrückt werden, bis sie außer Eingriff mit den Rastnocken 14a und in die in den Fig. 11/12 gezeigte Öffungsstellung gelangen.

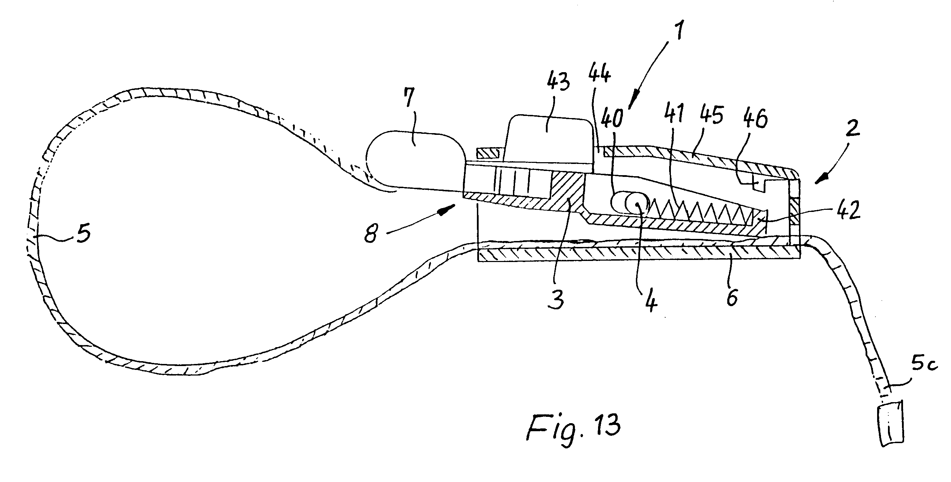

Während bei den vorstehend beschriebenen Ausführungsbeispielen die Öffnungsbetätigung von der voraufgehenden Lösebetätigung seitens der Bedienungsperson nicht oder bestenfalls durch einen unterschiedlichen Widerstand (Druckpunkt) der beteiligten Betätigungsorgane zu unterscheiden ist, so daß bei unsensibler Betätigung das Öffnen zu rasch auf das Lösen folgt, sehen die in den Fig. 13 bis 16 veranschaulichten Ausführungsbeispiele eine Sperre vor, welche die Betätigung zunächst auf das Erreichen der Lösestellung begrenzt und ein kurzzeitiges Nachlassen des Betätigungsdruckes verlangen, damit die weitere Betätigung in die Öffnungsstellung - durch neuerlichen Betätigungsdruck - vorgenommen werden kann.

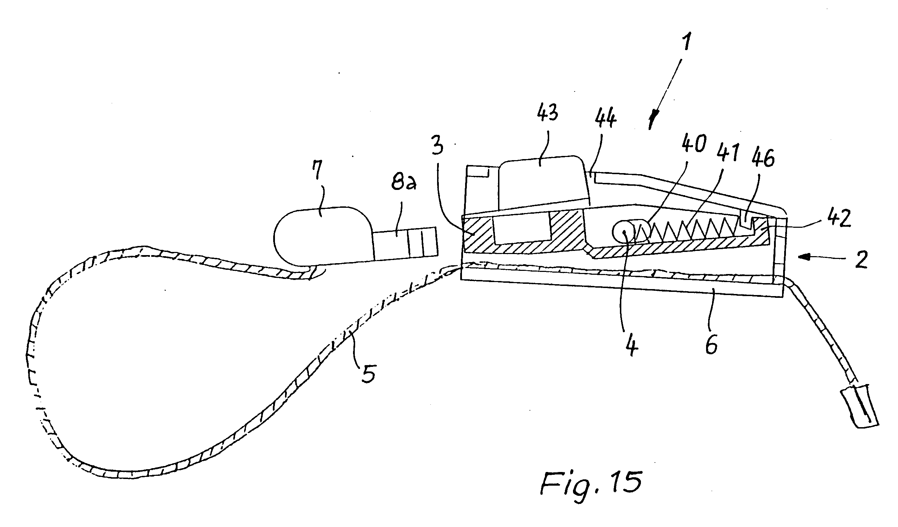

Die Klemmwippe 3 ist mittels seitlicher Langlöcher 40 auf der im Gehäuse 2 fest gehaltenen Achse 4 nicht nur schwenkbar gelagert, sondern auch in Längsrichtung (Längsachse 19 in Fig. 4) begrenzt verschieblich geführt. Eine Feder 41, die sich an der Achse 4 abstützt, wirkt auf einen abgekröpften Schenkel 42 der Klemmwippe 3 und sucht diese in ihre Öffnungsstellung zu drücken, in welcher die Langlöcher 40 mit ihren in den Zeichnungen linken Enden an der Achse 4 anliegen (Fig. 15).

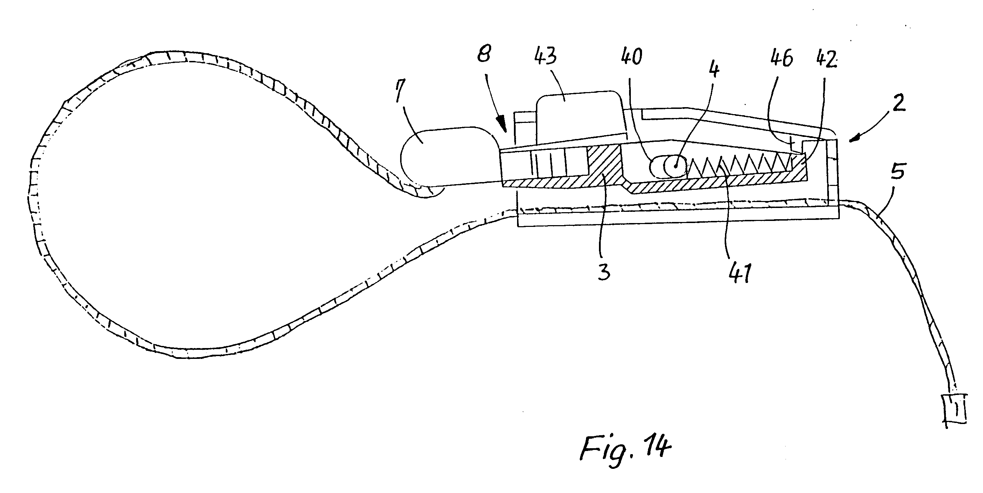

Beim Anlegen der Abschnürvorrichtung wird - nach Schließen des Rastverschlusses 8 - die Klemmwippe 3 infolge der Abschnür-Zugkraft im Band 5 jedoch gegen die Kraft der Feder 41 in ihre in Fig. 13 dargestellte Lage gezogen, in der die in den Zeichnungen rechten Enden der Langlöcher 40 an der Achse 4 anliegen. Wird dann zum Lösen der Abschnürung Druck auf die Drucktaste 43 ausgeübt, welche durch eine Öffnung 44 des oberseitig mit einem Deckel 45 geschlossenen Verschlußgehäuses 2 ausgeübt, dann schwenkt die Klemmwippe 3 aus ihrer in Fig. 13 dargestellten Abschnürstellung in die in Fig. 14 dargestellte Lösestellung. Dabei schlägt der abgekröpfte Schenkel 42 an einem Anschlag 46 am Deckelabschnitt 45 an. Erst wenn der Druck auf die Drucktaste 43 vorübergehend unterbrochen wird, kann die Feder 41 die Klemmwippe 3 weiter in die in Fig. 15 dargestellte Öffnungsstellung schwenken, weil jetzt der Ansatz 42 den Anschlag 46 passieren kann. Dabei wird - in einer der vorstehend beschriebenen Weisen - der Rastverschluß 8 geöffnet.

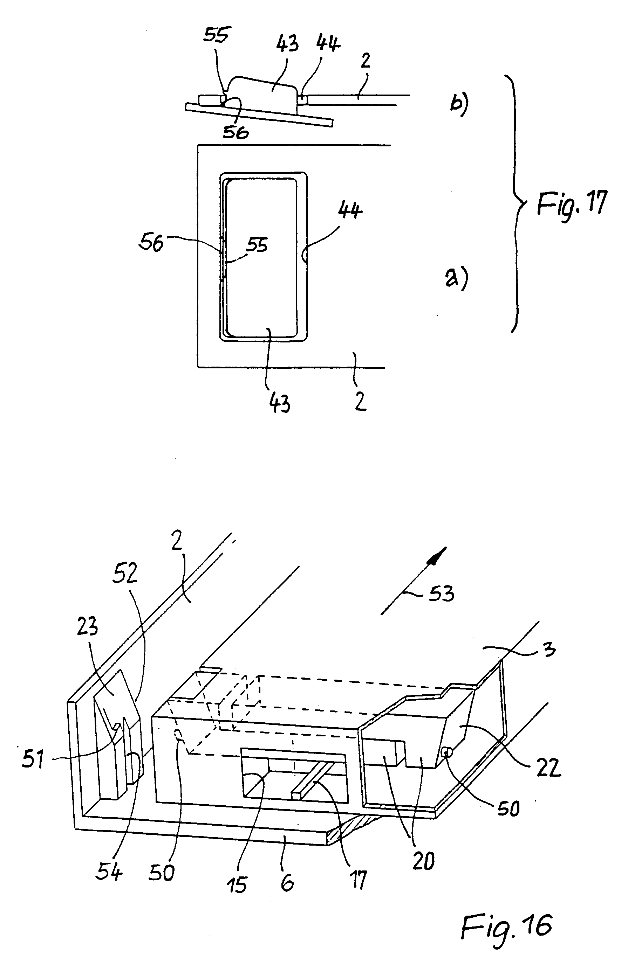

Fig. 16 zeigt eine abgewandelte Ausführungsform der als "Fangnest" ausgebildeten Sperre gegen einen absatzlosen Übergang von der Lösebetätigung in die Öffnungsbetätigung. Hier sind die anhand der Fig. 3 - 6 beschriebenen Keilflächen 22, 23 zusätzlich mit in Längsrichtung gegeneinander versetzten Sperrgliedern versehen. Die an den Schiebegliedern 20 angebrachten Zapfen 50 werden am Ende der Lösebetätigung der Klemmwippe 3 in taschenförmigen Vertiefungen 51 in den die Keilflächen 23 aufweisenden Öffnungsgliedern 52 gefangen. Läßt der Druck auf die Klemmwippe im Löse- und Öffnungssine kurzzeitig nach, wird diese (von einer der Feder 41 entsprechenden Feder) in Richtung des Pfeiles 53 gezogen, so daß bei erneuter Betätigung die Zapfen 50 in Nuten 54 eintauchen und die Klemmwippe weiter im Öffnungssinne geschwenkt werden kann, wodurch das Zusammenwirken der Keilflächen 22 und 23 zum Öffnen des Rastverschlusses führt.

Fig. 17 zeigt schematisch die Anordnung von Vorsprüngen 55, 56 an der Drucktaste 43 (des Ausführungsbeispiels der Fig. 13 - 15) bzw. der sie umgebenden Öffnung 44 im Deckel des Verschlußgehäuses 2. Beim Lösevorgang passiert der Vorsprung 55 den Vorsprung 56 und erzeugt dabei ein Knackgeräusch, welches der Bedienungsperson das Erreichen der Lösestellung signalisiert.

## Patentansprüche

1. Abschnürvorrichtung für Körperteile, insbesondere Venenstauer, mit einem Gehäuse (2), in dem eine Klemmwippe (3) schwenkbar gelagert ist, welche in einer Klemmstellung mit ihrem einen Ende (11) ein das Gehäuse durchsetzendes Band (5) klemmend gegen eine Gehäusewand (6) drückt und in einer entgegengesetzt gerichteten Lösestellung das Band freigibt, wobei das in der Abschnürlage unter Spannung stehende Band die Klemmwippe in ihre Klemmstellung schwenkt, während durch manuellen Druck auf die Klemmwippe diese in die Lösestellung schwenkbar ist, sowie mit einem lösbaren Rastverschluß (8) zwischen dem anderen Ende der Klemmwippe (3) und einer Kappe (7) am gehäuseseitigen Ende des Bandes (5),
**gekennzeichnet durch** mindestens ein mechanisches Übertragungsglied (22, 23; 26, 28; 31), welches bei direkter oder indirekter Ausübung eines Betätigungsdruckes auf die Klemmwippe (3) zunächst die Schwenkung der Klemmwippe in ihre Lösestellung zuläßt und nach der Freigabe des Bandes (5) den Rastverschluß (8) löst.

2. Vorrichtung nach Anspruch 1, dadurch gekennzeichnet, daß beim Schwenken der Klemmwippe (3) aus ihrer Klemmstellung das Übertragungsglied (22, 23; 26, 28; 31) erst in der Lösestellung der Klemmwippe in Wirkverbindung zum Rastverschluß (8) steht und diesen - bei weiterem Schwenken der Klemmwippe bis in eine Öffnungsstellung - löst.

3. Vorrichtung nach Anspruch 1 oder 2, dadurch gekennzeichnet, daß das Übertragungsglied (22, 23; 26, 28; 31) dem Betätigungsdruck beim Öffnen des Rastverschlusses (8) einen größeren Widerstand entgegensetzt als zur Freigabe des Bandes (5) an der Klemmwippe (3) als Druckpunkt zu überwinden ist.

4. Vorrichtung nach Anspruch 3, dadurch gekennzeichnet, daß die Schließkraft mindestens einer Feder, die den Rastverschluß (8) in seine Schließstellung drückt, mit einer größeren Kraftkomponente der Öffnungsbetätigung entgegenwirkt als das Band (5) in seiner Abschnürstellung auf die Klemmwippe (3) im Klemmsinne ausübt.

5. Vorrichtung nach Anspruch 1, dadurch gekennzeichnet, daß eine Sperre (42, 46; 50, 51) vorgesehen ist, die die Wirksamkeit des Übertragungsgliedes (22, 23; 26, 28; 31) bis zu einer Unterbrechung des Betätigungsdruckes nach der Freigabe des Bandes (5) blockiert.

6. Vorrichtung nach Anspruch 5, dadurch gekennzeichnet, daß die Schwenklager (4) der Klemmwippe (3) im Gehäuse (2) längsverschieblich sind (40) und das Band (5) in seiner Abschnürlage die Klemmwippe (3) gegen die Kraft einer Feder (41) in eine erste Endstellung zieht, in der bei Druckbetätigung der Klemmwippe (3) ein Anschlag (46; 51) deren Schwenkbewegung auf das Erreichen der Lösestellung begrenzt, bis nach Freigabe des Bandes (5) die Feder (41) die Klemmwippe (3) in ihre zweite Endstellung bewegt, in der sie in ihre Öffnungsstellung geschwenkt werden kann und dabei den Rastverschluß (8) löst.

7. Vorrichtung nach Anspruch 6, dadurch gekennzeichnet, daß der Anschlag (46; 51) einen abgekröpften Abschnitt hat, der die Längsbewegung der Klemmwippe (3) unter der Federkraft verhindert, bis die Klemmwippe in eine Freigabestellung zurückschwenkt.

8. Vorrichtung nach mindestens einem der vorhergehenden Ansprüche, dadurch gekennzeichnet, daß das Übertragungsglied aus Schräg- oder Keilflächen (22, 23; 26, 27; 23a) besteht, durch welche die Rastpartner des Rastverschlusses (8) im Öffnungssinne betätigbar sind.

9. Vorrichtung nach Anspruch 8, dadurch gekennzeichnet, daß die Schräg- oder Keilflächen (22, 23; 23a) so ausgebildet und/oder angeordnet sind, daß sie erst nach Erreichen der Lösestellung des Bandes (5) in Eingriff kommen.

## Claims

1. A constricting device for body parts, in particular a means of constricting veins, having a housing (2) in which there is pivotally mounted a clamping rocker (3) which, in a clamping position, by means of its one end (11) presses a tape (5) passing through the housing in clamping manner against a housing wall (6) and, in an oppositely directed release position, frees the tape, where the tape, which is under tension in the constricting condition, pivots the clamping rocker into its clamping position, while manual pressure on the clamping rocker enables the latter to be pivoted into the release position, and having a releasable latching closure (8) between the other end of the clamping roller (3) and a cap (7) at the housing end of the tape (5), characterized by at least one mechanical transfer member (22, 23; 26, 28; 31) which, when an actuating pressure is exerted directly or indirectly on the clamping rocker (3), first allows the clamping rocker to pivot into its release position and, once the tape (5) has been freed, releases the latching closure (8).

2. A device according to Claim 1, characterized in that when the clamping rocker (3) pivots out of its clamping position the transfer member (22, 23; 26, 28; 31) comes into operative connection with the latching closure (8) only when the clamping rocker is in the release position, and releases this latching closure (8) when the clamping rocker pivots further, into an opening position.

3. A device according to Claim 1 or 2, characterized in that the transfer member (22, 23; 26, 28; 31) offers a greater resistance to the actuating pressure on opening the latching closure (8) than that which has to be overcome as a pressure point to release the tape (5) at the clamping rocker (3).

4. A device according to Claim 3, characterized in that the closing force of at least one spring pressing the latching closure (8) into its closed position opposes the actuation of opening with a greater component of force than the tape (5) exerts on the clamping rocker (3) in the direction of clamping, in its constricting position.

5. A device according to Claim 1, characterized in that a locking means (42, 46; 50, 51) is provided, which blocks the effectiveness of the transfer member (22, 23; 26, 28; 31) until the actuating pressure is interrupted after release of the tape (5).

6. A device according to Claim 5, characterized in that the pivot bearings (4) of the clamping rocker (3) are longitudinally displaceable (40) in the housing (2) and, in its constricting condition, the tape (5) draws the clamping rocker (3) in opposition to the force of a spring (41) into a first end position in which, when the clamping rocker (3) is actuated by pressure, a stop (46; 51) restricts the pivotal movement thereof to arrival at the release position until, on release of the tape (5), the spring (41) moves the clamping rocker (3) into its second end position, in which it can be pivoted into its opening position and thus releases the latching closure (8).

7. A device according to Claim 6, characterized in that the stop (46; 51) has a chamfered portion which prevents longitudinal movement of the clamping rocker (3) under the spring force until the clamping rocker pivots back into a freeing position.

8. A device according to at least one of the preceding claims, characterized in that the transfer member comprises sloping or wedge surfaces (22, 23; 26, 27; 23a) by means of which the latching partners of the latching closure (8) may be actuated in the direction of opening.

9. A device according to Claim 8, characterized in that the sloping or wedge surfaces (22, 23; 23a) are constructed and/or arranged such that they come into engagement only once the tape (5) has arrived at its release position.

## Revendications

1. Dispositif de ligature pour des parties du corps humain, en particulier un tourniquet, comprenant un boîtier (2), dans lequel est montée, de manière à pouvoir pivoter, une bascule de serrage (3), qui, dans une position de blocage, bloque avec l'une de ses extrémités (11) contre une paroi (6) du boîtier un ruban (5), qui traverse le boîtier, et libère le ruban dans une position de desserrage, orientée dans le sens opposé, le ruban mis sous tension en position de ligature faisant pivoter la bascule de serrage dans la position de blocage, alors qu'une pression manuelle sur la bascule de serrage repositionne cette dernière en position de desserrage, ainsi qu'un système de verrouillage à encoches (8) situé entre l'autre extrémité de la bascule de serrage (3) et une calotte (7) à l'extrémité du ruban (5) du côté du boîtier,
caractérisé en ce qu'au moins un organe de transfert mécanique (22, 23 ; 26, 28 ; 31), qui, par une pression de commande exercée directement ou indirectement sur la bascule de serrage (3), permet d'abord le pivotement de la bascule de serrage vers sa position de desserrage puis débloque le système de verrouillage à encoches (8) après le déblocage du ruban (5).

2. Dispositif selon la revendication 1, caractérisé en ce que, lors du pivotement de la bascule de serrage (3) hors de sa position de blocage, une liaison active entre l'organe de transfert (22, 23 ; 26, 28 ; 31) et le système de verrouillage à encoches (8) est obtenue uniquement lorsque la bascule de serrage (3) a atteint sa position de desserrage et débloque le système de verrouillage - au cas où la bascule de serrage continue à pivoter jusqu'à sa position d'ouverture.

3. Dispositif selon la revendication 1 ou 2, caractérisé en ce que l'organe de transfert (22, 23 ; 26, 28 ; 31) oppose à la pression de commande, exercée pour ouvrir le système de verrouillage à encoches (8), une plus forte résistance que celle nécessaire en tant que point de pression pour débloquer le ruban (5) de la bascule de serrage (3).

4. Dispositif selon la revendication 3, caractérisé en ce que l'effort de fermeture d'au moins un ressort, qui bloque le système de verrouillage à encoches (8) dans sa position de fermeture, réagit avec une plus grande composante de force du mouvement d'ouverture que celle exercée par le ruban (5) dans sa position de ligature sur la bascule de serrage (3), afin d'être bloqué.

5. Dispositif selon la revendication 1, caractérisé en ce qu'il est prévu de monter un taquet (42, 46 ; 50, 51), qui bloque l'action de l'organe de transfert (22, 23 ; 26, 28 ; 31) jusqu'à l'interruption de la pression de commande après le déblocage du ruban (5).

6. Dispositif selon la revendication 5, caractérisé en ce que les paliers de pivotement (4) de la bascule de serrage (3) dans le boîtier (2) peuvent être déplacés dans le sens longitudinal (40) et en ce que le ruban (5) dans sa position de ligature exerce une traction sur la bascule de serrage (3) à l'encontre de la force exercée par un ressort (41) dans une première position finale, dans laquelle, lors d'une pression de commande exercée sur la bascule de serrage (3), un taquet (46 ; 51) limite le mouvement de pivotement de cette dernière jusqu'à atteindre la position de desserrage, jusqu'à ce qu'après le déblocage du ruban (5) le ressort (41) fasse pivoter la bascule de serrage (3) dans sa deuxième position finale, dans laquelle elle peut pivoter vers sa position d'ouverture, et débloque en même temps le système de verrouillage à encoches (8).

7. Dispositif selon la revendication 6, caractérisé en ce que le taquet (46 ; 51) est muni d'une partie coudée, qui empêche le déplacement longitudinal de la bascule de serrage (3) sous l'effet de la force exercée par le ressort, jusqu'à ce que la bascule de serrage soit replacée dans sa position de déblocage de départ.

8. Dispositif selon l'une quelconque des revendications précédentes, caractérisé en ce que l'organe de transfert est formé de surfaces obliques ou de surfaces tronconiques (22, 23 ; 26, 27 ; 23a), dans lesquelles les systèmes destinés à recevoir les encoches du système de verrouillage à encoches (8) peuvent être actionnés afin d'ouvrir ledit système de verrouillage.

9. Dispositif selon la revendication 8, caractérisé en ce que les surfaces obliques ou les surfaces tronconiques (22, 23 ; 23a) sont conçues ou disposées de telle sorte qu'elles permettent l'engrènement seulement après que le ruban (5) a atteint sa position de desserrage.
